(19) Europäisches Patentamt / European Patent Office / Office européen des brevets

(11) **EP 3 603 509 A1**

(12) **EUROPEAN PATENT APPLICATION**

(43) Date of publication:
**05.02.2020 Bulletin 2020/06**

(51) Int Cl.:
***A61B 5/145*** (2006.01)

(21) Application number: **19188731.4**

(22) Date of filing: **28.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2018 US 201816052675**

(71) Applicant: **Cnoga Medical Ltd.
3088900 Caesarea (IL)**

(72) Inventor: **SEGMAN, Yosef
3088900 Caesarea (IL)**

(74) Representative: **Kancelaria Eupatent.pl Sp. z.o.o
Ul. Kilinskiego 185
90-348 Lodz (PL)**

(54) **SYSTEM AND METHOD FOR CONTROLLING BLOOD GLUCOSE USING PERSONALIZED HISTOGRAMS**

(57) A method of control of diabetes in a patient, comprising: measuring, using a non-invasive or invasive glucose measuring device, momentary glucose levels of the patient at multiple times a day during an initial time period either continuously or at discrete times, the initial time period being at least a week; recording the momentary glucose levels in a memory in communication with the device, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading; constructing, using one or more processors in communication with the device, current curvilinear probability density functions of occurrences of the momentary glucose levels throughout the initial time period and displaying the curvilinear probability density functions on a screen; determining, using one or more processors, a quantitative assessment of at least one of the following current momentary markers during the measurement period from the momentary glucose levels recorded and the optional information: (i) Insulin Resistance (IR), (ii) Glucose Variability (GV), (iii) Glucose Severity Index (GSI) or (iv) Glucose Burden (GB), and constructing, using the one or more processors, a personalized target curvilinear probability density function for the patient based on the current curvilinear probability density functions.

Figure 1A

**Figure 1B**

**Figure 1C**

**Description**

FIELD AND BACKGROUND OF THE INVENTION

The invention relates to apparatuses and methods for controlling blood glucose using personalized histograms.
Diabetes is a large and growing global health concern. The World Health Organization declared diabetes as a global epidemic due to the increasing prevalence of the disease in the last few decades among all populations around the globe. The number of diabetes patients is estimated to hit 36 million by 2030. This burden requires continued improvement in diabetes care.

SUMMARY OF THE PRESENT INVENTION

One aspect of the invention is a method of control of diabetes in a patient, comprising measuring, using a non-invasive or invasive glucose measuring device, momentary glucose levels of the patient at multiple times a day during an initial time period either continuously or at discrete times, the initial time period being at least a week; recording the momentary glucose levels in a memory in communication with the device, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading; constructing, using one or more processors in communication with the device, current curvilinear probability density functions of occurrences of the momentary glucose levels throughout the initial time period and displaying the curvilinear probability density functions on a screen; determining, using one or more processors, a quantitative assessment of at least one of the following current momentary markers during the measurement period from the momentary glucose levels recorded and the optional information: (i) Insulin Resistance (IR), (ii) Glucose Variability (GV), (iii) Glucose Severity Index (GSI) or (iv) Glucose Burden (GB), and constructing, using the one or more processors, a personalized target curvilinear probability density function for the patient based on the current curvilinear probability density functions.
In some embodiments, the method further comprises verifying a success of a personal treatment by at least one of (i) verifying improvement of one or more of the IR, GV and the GB compared to a respective IR, GV and GB of a previous period and (ii) verifying reduction of a level of GSI compared to a level of GSI in a previous period, and by verifying a match of a new curvilinear probability density function constructed based on momentary glucose levels measured after the personal treatment with the target curvilinear probability density function.
In some embodiments, the method further comprises determining, using one or more processors, the quantitative assessment of the at least one of the following current momentary markers during the measurement period from the momentary glucose levels recorded and the optional information: (i) insulin resistance (IR), (ii) glucose severity index (GSI), (iii) glucose burden (GB) and (iv) glucose variability and constructing the personalized target curvilinear probability density function for the patient, the target curvilinear probability density function configured to improve at least one of (i) the IR, (ii) the GSI, (iii) the GB and (iv) the GV of the patient.
In some embodiments, the method further comprises constructing the curvilinear probability density functions from glucose histograms.
In some embodiments, the curvilinear probability density functions are the glucose histograms.
In some embodiments, the method further comprises constructing glucose histograms that represent the occurrences of the momentary glucose levels throughout only certain hours of day over a time period, time period spanning all or part of the initial time period.
In some embodiments, the suggested diabetes treatment is at least one of the following: diet, rest, physical activity, insulin and drugs.
In some embodiments, the personalized target curvilinear probability density function reflects a severity of diabetes that is less severe than the severity reflected by the shape of the current curvilinear probability density functions during the initial time period for the same patient.
In some embodiments, the personalized target curvilinear probability density function is configured to improve at least one of the following quantitative assessments: GV, GSI, IR and GB of the patient, wherein improving GSI means reducing a frequency of glucose above a predefined amount level as well as improving the glucose burden and shifting the target histogram to have a single peak closer to a normal glucose value, improving GB means reducing GB level for instances of hyperglycemia and increasing GB level for instances of hypoglycemia and improving IR means improving absorption of glucose in the cells of the patient's body.
In some embodiments, the personalized target curvilinear probability density function reflects a first stage of personal improvement that precedes a second stage and any further stages of personal improvement designed by a personalized targeted curvilinear density function.
In some embodiments, the curvilinear probability density functions include the optional information as a look up table or as an additional axis. In some embodiments, the optional information includes the time of recent food eating. In some embodiments, the optional information includes the physical activity. In some embodiments, the optional information includes the diabetes treatment.

In some embodiments, the method further comprises determining, by the one or more processors, a quantitative measure of an extent to which the target curvilinear shape of the patient's curvilinear probability density function was realized during the second time period.

In some embodiments, the target curvilinear probability density function is a Gaussian-like shape.

In some embodiments, the method further comprises monitoring a response to a diabetes treatment including (i) monitoring, and recording a quantitative extent of, a change over time in the shape of the curvilinear probability density function in a direction of being more Gaussian; and (ii) monitoring, and recording a quantitative extent of, a shift of the curvilinear probability density function reflecting a lowering of a glucose level at which one or more peaks appear in the curvilinear probability density function.

In some embodiments, the method further comprises outputting at least one of (i) a type of diabetes, an existence of hyperglycemia, an existence of hypoglycemia, and a degree of severity of the diabetes; (ii) a tentative diagnostic suggestion; and (iii) a tentative treatment suggestion.

In some embodiments, the measuring is performed using a non-invasive glucose measuring device.

In some embodiments, the multiple times of each day in the initial time period includes at least a first time before a meal and a second time after a meal.

In some embodiments, the constructing, using the one or more processors, of the personalized target curvilinear probability density function for the patient is also based on the quantitative assessment.

Another aspect of the invention is a system for controlling blood glucose of a patient by using personalized histograms, comprising a non-invasive or invasive glucose measuring device configured to either continuously or at discrete times measure glucose levels of the patient including at multiple times a day during an initial time period, the initial time period being at least a week; a storage memory for recording the momentary glucose levels, the memory in communication with the device, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading; one or more processors either in the glucose measuring device or external to the device and in communication with the glucose measuring device and the memory, for constructing, current curvilinear probability density functions of occurrences of the momentary glucose levels throughout the initial time period and displaying the curvilinear probability density functions on a screen; the one or more processors configured to determine a quantitative assessment of at least one of the following current momentary markers during the measurement period from the momentary glucose levels recorded and the optional information: (i) Insulin Resistance (IR), (ii) Glucose Variability (GV), (iii) Glucose Severity Index (GSI) or (iv) Glucose Burden (GB), and the one or more processors configured to also construct a personalized target curvilinear probability density function for the patient based on the current curvilinear probability density functions.

In some embodiments, the one or more processors are configured to verify a success of a personal treatment by at least one of (i) verifying improvement of one or more of the IR, GV and the GB compared to a respective IR, GV and GB of a previous period and (ii) verifying reduction of a level of GSI compared to a level of GSI in a previous period, and by verifying a match of a new curvilinear probability density function constructed based on momentary glucose levels measured after the personal treatment with the target curvilinear probability density function.

In some embodiments, the one or more processors are further configured to determine the quantitative assessment of at least one of the following current momentary markers during the measurement period from the momentary glucose levels recorded and the optional information: (i) insulin resistance (IR), (ii) glucose severity index (GSI), (iii) glucose burden (GB) and (iv) glucose variability and to construct the personalized target curvilinear probability density function for the patient, the target curvilinear probability density function configured to improve at least one of (i) the IR, (ii) the GSI, (iii) the GB and (iv) the GV of the patient.

In some embodiments, the one or more processors are configured to construct the curvilinear probability density functions from glucose histograms.

In some embodiments, the curvilinear probability density functions are the glucose histograms.

In some embodiments, the one or more processors are configured to construct glucose histograms that represent the occurrences of the momentary glucose levels throughout only certain hours of day over a time period, time period spanning all or part of the initial time period.

In some embodiments, the suggested diabetes treatment is at least one of the following: diet, rest, physical activity, insulin and drugs.

In some embodiments, the personalized target curvilinear probability density function reflects a severity of diabetes that is less severe than the severity reflected by the shape of the current curvilinear probability density functions during the initial time period for the same patient.

In some embodiments, the personalized target curvilinear probability density function is configured to improve at least one of the following quantitative assessments: GV, GSI, IR and GB of the patient, wherein improving GSI means reducing a frequency of glucose above a predefined amount level as well as improving the glucose burden and shifting the target histogram to have a single peak closer to a normal glucose value, improving GB means reducing GB level for instances of hyperglycemia and increasing GB level for instances of hypoglycemia and improving IR means improving absorption of glucose in the cells of the patient's body.

In some embodiments, the personalized target curvilinear probability density function reflects a first stage of personal improvement that precedes a second stage and any further stages of personal improvement designed by a personalized targeted curvilinear density function.

In some embodiments, the curvilinear probability density functions include the optional information as a look up table or as an additional axis. In some embodiments, the optional information includes the time of recent food eating. In some embodiments, the optional information includes the physical activity. In some embodiments, the optional information includes the diabetes treatment.

In some embodiments, the one or more processors are configured to determine a quantitative measure of an extent to which the target curvilinear shape of the patient's curvilinear probability density function was realized during the second time period.

In some embodiments, the target curvilinear probability density function is a Gaussian-like shape.

In some embodiments, the one or more processors are configured to monitor a response to a diabetes treatment including (i) monitoring, and recording a quantitative extent of, a change over time in the shape of the curvilinear probability density function in a direction of being more Gaussian; and (ii) monitoring, and recording a quantitative extent of, a shift of the curvilinear probability density function reflecting a lowering of a glucose level at which one or more peaks appear in the curvilinear probability density function.

In some embodiments, the one or more processors are configured to generate an output of at least one of (i) a type of diabetes, an existence of hyperglycemia, an existence of hypoglycemia, and a degree of severity of the diabetes; (ii) a tentative diagnostic suggestion; and (iii) a tentative treatment suggestion.

In some embodiments, the multiple times of each day in the initial time period includes at least a first time before a meal and a second time after a meal.

In some embodiments, the constructing, using the one or more processors, of the personalized target curvilinear probability density function for the patient is also based on the quantitative assessment.

These and other features, aspects and advantages of the present invention will become better understood with reference to the following drawings, descriptions and claims.

BRIEF DESCRIPTION OF THE DRAWINGS

Various embodiments are herein described, by way of example only, with reference to the accompanying drawings, wherein:

Figure 1A represents the glucose distribution of a healthy individual with an average of 92mg/dL for use in accordance with one embodiment of the invention;

Figure 1B represents the glucose distribution of an uncontrolled type 2 diabetic, with an average of 256 mg/dL for use in accordance with one embodiment of the invention;

Figure 1C represents the glucose distribution of a labile type 1 diabetic, with an average of 144 mg/dL for use in accordance with one embodiment of the invention;

Figure 2A represents glucose histograms of actual patients (A-D) and a box and whiskers plot for use in accordance with one embodiment of the invention;

Figure 2B represents glucose histograms of actual patients (A-D) and a box and whiskers plot for use in accordance with one embodiment of the invention;

Figure 2C represents glucose histograms of actual patients (A-D) and a box and whiskers plot for use in accordance with one embodiment of the invention;

Figure 2D represents glucose histograms of actual patients (A-D) and a box and whiskers plot for use in accordance with one embodiment of the invention;

Figure 3A shows three representative patients with very different glucose histogram but the same HBA1C level of 6.5% and a box and whiskers plot;

Figure 3B shows three representative patients with very different glucose histogram but the same HBA1C level of 8.8% to 9% and a box and whiskers plot;

Figure 4A shows glucose histograms of a type 2 diabetes patient and a box and whiskers plot for use in accordance with one embodiment of the invention;

Figure 4B shows glucose histograms of a type 2 diabetes patient and a box and whiskers plot for use with one embodiment of the invention;

Figure 5 represents glucose histograms of a prototypical type 2 diabetes patient as he improves his glycemic control in accordance with one embodiment of the invention;

Figure 6A represents glucose histograms of a type 2 diabetes patient divided by times of the day, encompassing 350 measurements over two months for use with an embodiment of the invention;

Figure 6B depicts the same data as 6A, which has been binned into 2 hour slots for use with an embodiment of the invention;

Figure 6C shows the same data as 6A, with time binned into 15 minute slots and the glucose levels binned into 8 steps for use with an embodiment of the invention;

Figure 6D shows the same data as 6A wherein the Glucose distribution of the type 2 diabetes patient is presented by stacked histograms, color coded by different hours during the day for use with an embodiment of the invention;

Figure 7A is a flow chart showing a method, in accordance with one embodiment of the invention;

Figure 7B is a flow chart showing a method, in accordance with one embodiment of the invention;

Figure 8 is a current curvilinear probability density functions of occurrences of the glucose levels throughout the initial time period constructed in accordance with one embodiment of the invention;

Figure 9 is a personalized target curvilinear probability density function for the patient constructed based on the current glucose histogram or the current curvilinear probability density functions, the optional information and the determinations of insulin resistance, glucose variability and glucose burden as a first stage of personal improvement in accordance with one embodiment of the invention;

Figure 10 is a personalized target curvilinear probability density function for the patient constructed based on the glucose histogram or the current curvilinear probability density functions, the optional information and the determinations of insulin resistance, glucose variability and glucose burden as a second stage of personal improvement in accordance with one embodiment of the invention;

Figure 11 shows a current curvilinear probability density functions of occurrences of the glucose levels throughout the initial time period and showing the time zone within the day constructed in accordance with one embodiment of the invention;

Figure 12 shows a personalized target curvilinear probability density function for the patient constructed based on the current curvilinear probability density functions, the optional information and the determinations of insulin resistance, glucose variability and glucose burden as a first stage of personal improvement in accordance with one embodiment of the invention;

Figure 13 shows a personalized target curvilinear probability density function for the patient constructed based on the current curvilinear probability density functions, the optional information and the quantitative assessments of at least one of insulin resistance, glucose variability, glucose severity index and glucose burden as a second stage of personal improvement in accordance with one embodiment of the invention;

Fig. 14 is a schematic illustration of a system in accordance with one embodiment of the invention;

Fig. 15 (Example 1) is a histogram whose shape is considered to be that of a healthy individual;

Fig. 16 (Example 2) is a histogram whose shape is considered to be that of an uncontrolled type 2 diabetic individual;

Fig. 17 (Example 3) is a histogram whose shape is considered as a mid type 2 level diabetic or as being controlled under appropriate treatment in accordance with one embodiment of the invention; and

Fig. 18 (Example 4) is an unstable type 1 individual who fluctuates between hypo glycemia and hyper glycemia.

DETAILED DESCRIPTION OF THE INVENTION

The following detailed description is of the best currently contemplated modes of carrying out the invention. The description is not to be taken in a limiting sense, but is made merely for the purpose of illustrating the general principles of the invention, since the scope of the invention is best defined by the appended claims.

The current standard of care for detecting and controlling diabetes status is frequently based on measuring HBA1C. However, as an average value, it doesn't give precise information about the patient glycemic behavior or give an indication on the individual patient's metabolism, among other problems. Applicant instead proposes analyzing the actual glycemic behavior using data from intensive self-monitoring. Although, the frequent self-monitoring of blood glucose has been shown to improve diabetes management, intensive self-monitoring using invasive procedures (i.e. finger lancets and glucose strips) is impractical on the long term. The use of continuous glucose monitors, which have been shown to improve outcomes, is an appealing alternative, but these monitors also have drawbacks such as having been tested mainly in type 1 diabetes, requiring high compliance to show significant benefit, biocompatibility problems, requiring the attachment of implantable sensors and having significant economic costs. Although non-invasive glucose measuring technologies are coming of age, among them the TensorTip Combo Glucometer (CoG) developed by Applicant Cnoga Medical Ltd. which allows patients to measure themselves with almost no discomfort and pain, as well as being able to do repeated, frequent measurements for intensive monitoring, obtaining the data is not enough, since the large amounts of data generated by frequent monitoring are not always interpreted and exploited properly, and physicians and patients alike can struggle to make practical use of it.

One embodiment of the invention is a novel way to analyze and interpret longitudinal glycemic data. According to one embodiment of the invention, an approach to glycemic control uses the insights gained from such an analysis. These two together can harness the data for improved clinical use in a way that is highly amenable to be integrated into informatics-assisted diabetes care.

The invention generally provides a system and method for controlling blood glucose in a patient using personalized histograms. The system and method utilize an invasive or noninvasive glucose measuring device that measures continuously or at discrete times or intervals (uniform or non-uniform) The system and method involve recording in a memory of the device, momentary glucose levels of the patient at multiple times of each day of an initial time period, for example at least a first time before meals and a second time after meals, the initial time period being at least a week. The recording may include optional information associated with the recent temporal glucose reading including one or more of the following information: date, time recent food eating, recent physical activity, diabetes treatment, body temperature, blood pressure, pulse, drugs including specialized drugs, associated with a recent temporal glucose reading. Using one or more processors, the system or method constructs current curvilinear probability density functions of occurrences of the glucose levels throughout the initial time period and, in some embodiments, this is displayed on a screen, printed or sent by electronic means. The optional information may be incorporated with the curvilinear glucose density function.

Using the one or more processors, in some embodiments a quantitative assessment of at least one of (or at least two of or at least three of) the following current momentary biomarkers is determined during the measurement period from the momentary glucose levels recorded and the optional information: (i) Insulin Resistance (IR), (ii) Glucose Variability (GV), (iii) Glucose Severity Index (GSI) or (iv) Glucose Burden (GB). Using the one or more processors, a personalized target curvilinear probability density function is constructed for the patient based on the current curvilinear probability density functions and optionally also based on the quantitative assessment. The glucose severity index is based on at least one of a diabetes type and a degree of severity of the patient's diabetes.

The method may also include verifying a success of a personal treatment by at least one of (i) verifying the improvement of one or more of the IR, GV and the GB compared to a respective IR, GV and GB of a previous period and (ii) verifying the reduction of a level of GSI compared to a level of GSI in a previous period, and by verifying a match of a new curvilinear probability density function constructed based on glucose levels data measured after the personal treatment with the target curvilinear probability density function. The personalized target curvilinear probability density function may be displayed on a screen, printed or sent by electronic means.

Diabetes is inherently linked to a patient's lifestyle. In addition, there is a large variability of individual metabolism. Due to the complexity of diabetes, it is beneficial to diagnose the different stages and subtypes of the disease, together with specific treatments that addresses the individual metabolic profiles and disease stages. In addition, the digitalization of diabetes care needs coupled to the patients' actual data and care specifics, can provide a relevant, positive feedback loop that encourages further engagement. Thus, the provision of patient level data emerges as a crucial point in the quest for better, personalized diabetes care, to be provided together with digital health technologies.

The principles and operation of a System and Method for Controlling Blood Glucose Using Personalized Histograms may be better understood with reference to the drawings and the accompanying description.

Today glycemia control is based on HbAlc known for glycated hemoglobin. HbAlc is a single biomarker representing periodic average blood glucose level. The suggested approach herein is based on a personal temporal curvilinear density histogram. A periodic temporal curvilinear glucose density histogram provides higher resolution of the glucose distribution instead of a single averaging glucose biomarker. Any existing home glucometer device may provide a periodic glucose curvilinear glucose density histogram shape. In accordance with one embodiment of the invention, the method has two major stages: An initial stage of measuring blood or tissue glucose levels during an initial period i.e. a week, two weeks, three weeks, a month, two months, etc., wherein the individual will monitor himself few times a day, i.e. 5 times, an optimal daily frequent glucose reading is eight times per day for a suggested period of two weeks. The initial stage will produce the initial temporal curvilinear density histogram shape.

In accordance with a second stage of treatment, based on the initial temporal curvilinear density glucose histogram shape, a future temporal curvilinear density glucose histogram is the target for a treatment. "A treatment" means diet, rest and physical activity, drugs or any combination thereof. The goal of the treatment is to shift the current temporal density glucose curvilinear histogram shape into the new shape indicative of personal improvement of blood or tissue glucose. In some cases, the current improvement would be a step for a next improvement based on second future temporal curvilinear glucose density histogram.

Given a Period of Time **P,** for example one week, two weeks, four weeks, three months, six months, etc., Let H represents the occurrence of glucose level **g** during the period of time **P**. For example H(100) represents how many times the glucose level 100gl/dL occur during the period of time P.

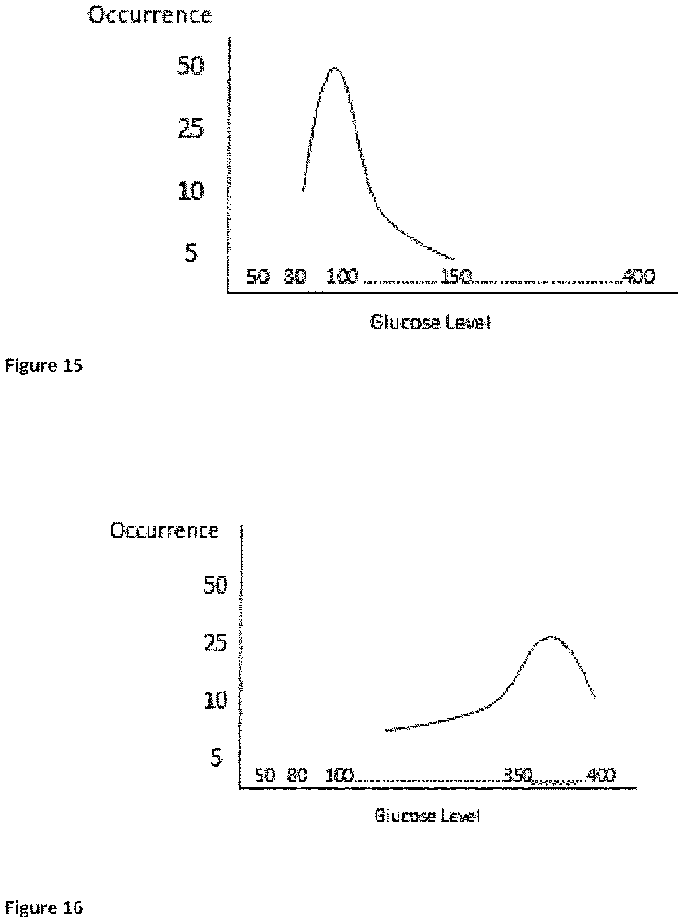

Example 1 (Fig. 15)

In Example 1 (Fig. 15), about 50 times of the measurements occur at 100gl/dL, about 10 times occur at 80gl/dL and about 5 times occur at 150gl/dL. This shape of Histogram may be considered to be that of a healthy individual.

Example 2 (Fig. 16)

In Example 2 (Fig. 16), a glucose level frequency of about 30 times occurs at 370gl/dL, while a frequency of below 10 times occurs at level 190gl/dL. This shape of histogram may be considered to be that of an uncontrolled type2 diabetic individual.

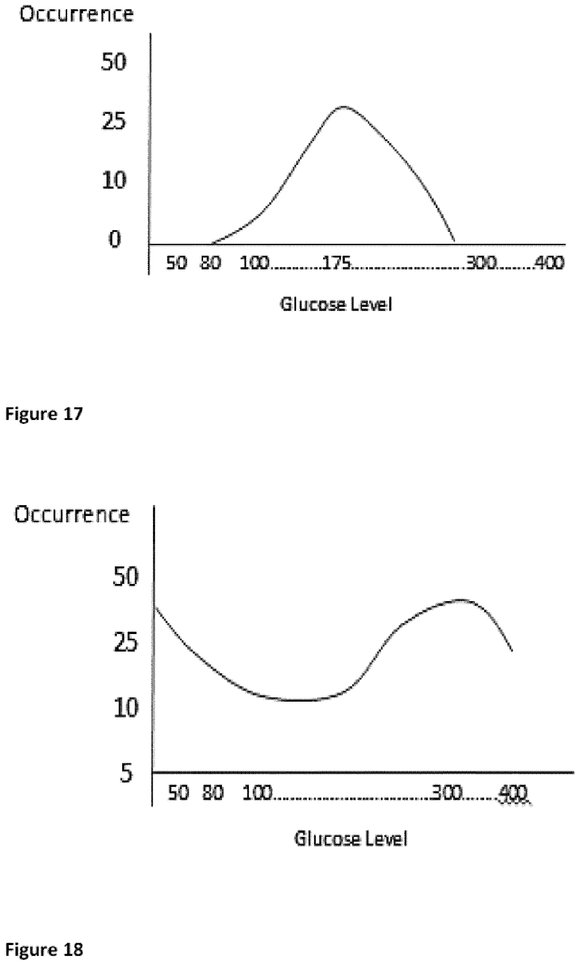

Example 3 (Fig. 17)

In Example 3 (Fig. 17), a glucose level frequency of about 40 times occurs at 175gl/dL, a frequency of about 7 times occurs at 100gl/dL and a frequency of about 10 times occurs at 265gl/dL. This shape of histogram may be considered as a mid type2 level diabetic or as being controlled under appropriate treatment resulting in histogram shape improvement from Example 2 (Fig. 16) to Example 3 (Fig. 17).

Example 4 (Fig. 18)

In Example 4 (Fig. 18), a frequency of about 40 times of the glucose level occurs below 50mg/dL and a frequency about 40 times occurs above 300mg/dL. This case represents unstable type 1 individual who fluctuates between hypo glycemia and hyper glycemia.

In order to better understand the glycemic behavior of the patients, one possible approach is to plot the glucose levels using a histogram in order to discern the underlying distribution of values. A glucose histogram is a function of the following form:

$$H(g) = H(g) + 1;$$

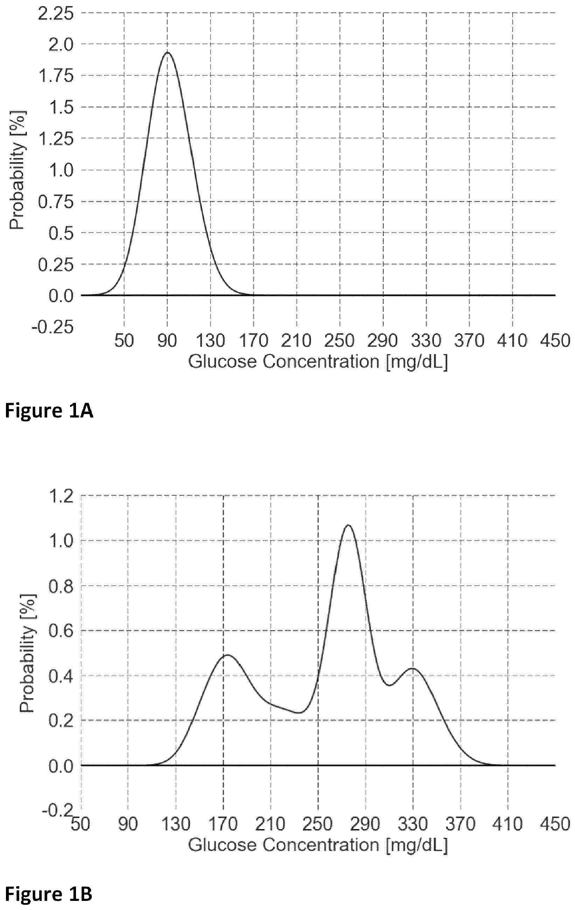

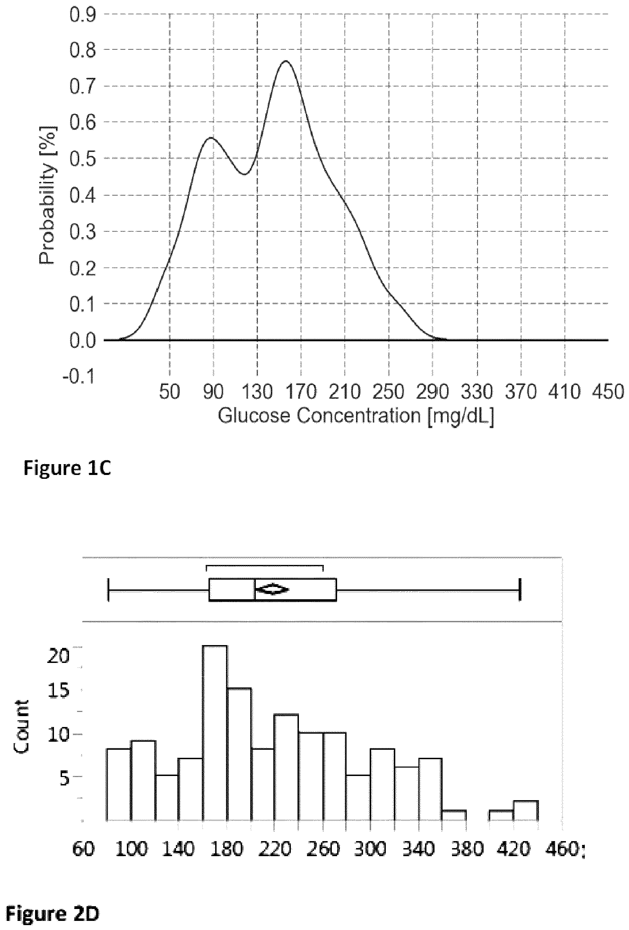

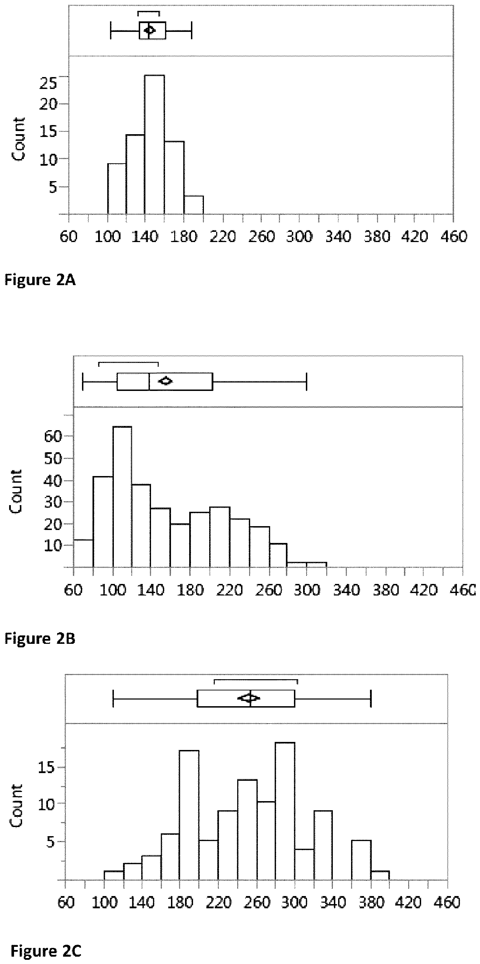

where g represents glucose value. The curvilinear probability density function and the shape or appearance of the graph are generated from the histogram. For example, Figure 1A represents the shape of a curvilinear probability density function generated from a histogram of an actual healthy individual. Figure 1B, in contrast, represents an actual severely unbalanced type 2 diabetic, with elevated glucose levels overall and a tri-modal distribution. Figure 1C represents an actual labile type 1 diabetic, who suffers from several episodes of both hypo- and hyper-glycemia (29% of the readings are below 80 md/dL and 8% of the readings are above 200 mg/dL). Figure 2A shows a histogram without generating the curvilinear shape of the previous examples. The distribution of glucose values that is relatively Gaussian in shape, with a mean of about 150 mg/dL and some values that are above 180 mg/dL; this patient has slightly elevated glucose levels overall but the distribution shape is not abnormal. Figure 2B shows a patient with a bimodal distribution, with the 1st peak having relatively normal levels and a 2nd peak around 210 mg/dL. Figure 2C shows a Gaussian distribution that is both broad, with a high variability, and shifted to the right with a mean around 250 mg/dL. Finally, Figure 2D shows an almost flat distribution of glucose levels. Evidently these patients have very different problems in their glycemic control; such detailed information cannot be observed from a single HBA1C level. Thus, the glucose histogram is a useful and intuitive way to describe and understand our patients' glycemic behavior.

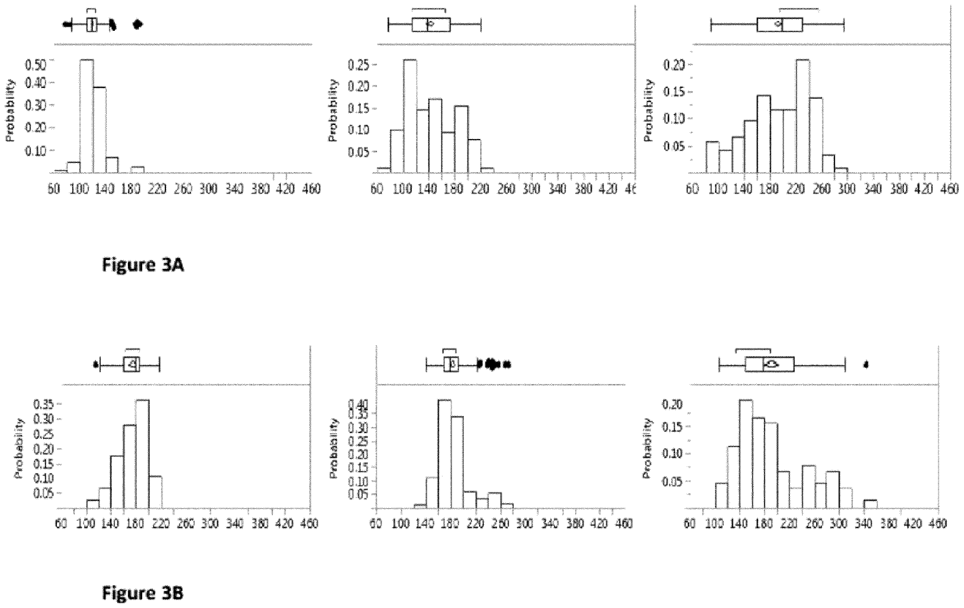

Different people glycosylate at different rates, and the use of HBA1C has a series of pitfalls including individual and ethnic variability in the correlation between glycemic burden and HBA1C levels, difficulty in the standardization of measurement methods and the effects of HB variants. For example, Figure 3A presents three different patients who had the same HBA1C level at the time of the trial, 6.5%, yet their glucose histograms are significantly different, with different shape, variability and glycemic burden. Similarly, Figure 3B shows three different patients with an HBA1C level of 8.8% to 9%.

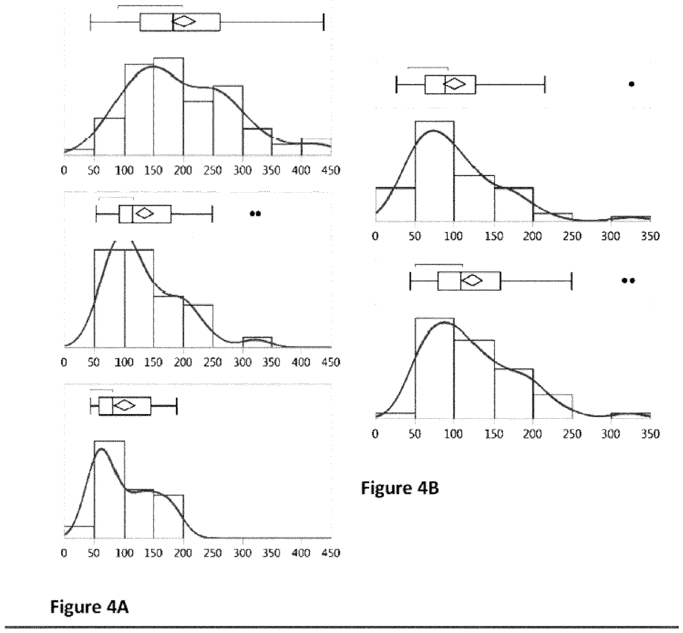

The glucose histogram is useful not only as a static measure of the patient's glycemic control, but also of his evolution over time, as shown in figure 4. Figure 4A illustrates the dramatic change in the glycemic behavior of a patient as she entered an intensive glucose control program with the help of the CoG, a change too fast to be reflected in the HBA1C. Figure 4B illustrates a second patient's histogram four months apart; although at first glance the two histograms look similar, the statistics clearly show a loosening of glycemic control, with all measures shifting 20 mg/dL higher.

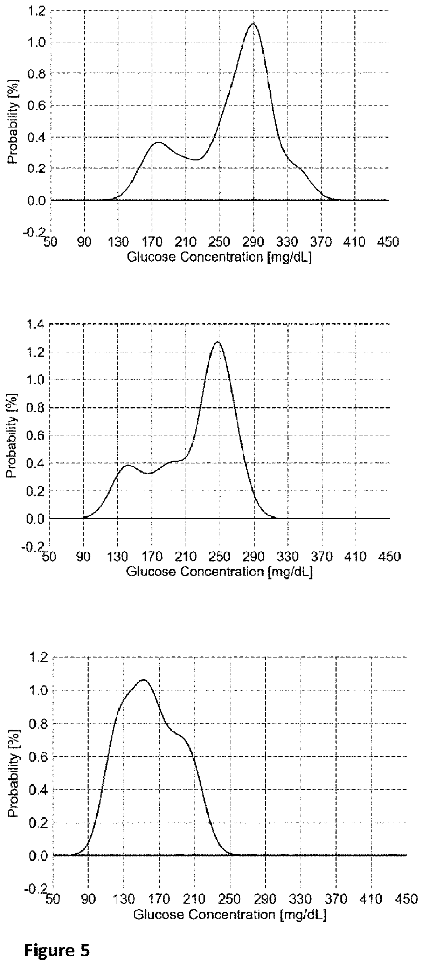

The glucose histogram allows us to use an alternative approach for glycemic control: to target specific features of the histogram, instead of using standard protocols or algorithms that are agnostic to our specific patient's characteristics. For example, Figure 5 shows the theoretical progression of an uncontrolled type 2 diabetes patient after beginning therapy. The first stage begins by targeting our interventions to changing the shape of the glucose histogram from a skewed distribution to a more normally shaped one, which also brings a general reduction of glucose levels. Once that is achieved, we then add an intervention aimed at shifting the whole distribution towards lower values, while avoiding the occurrence of hypoglycemia.

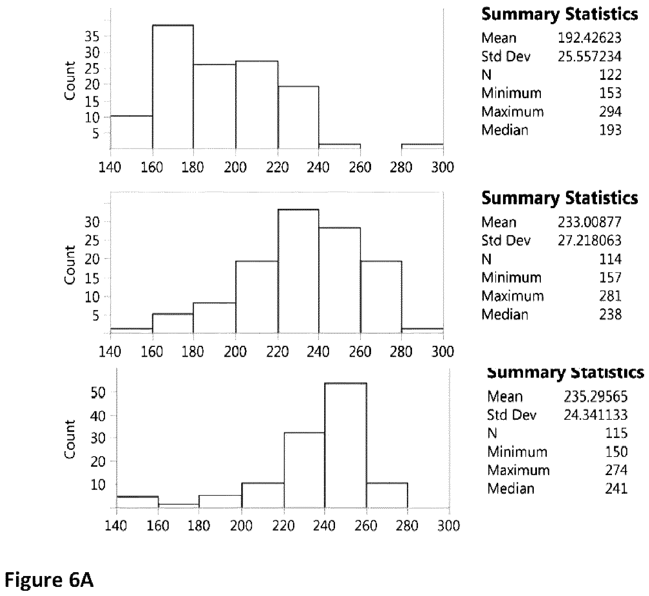

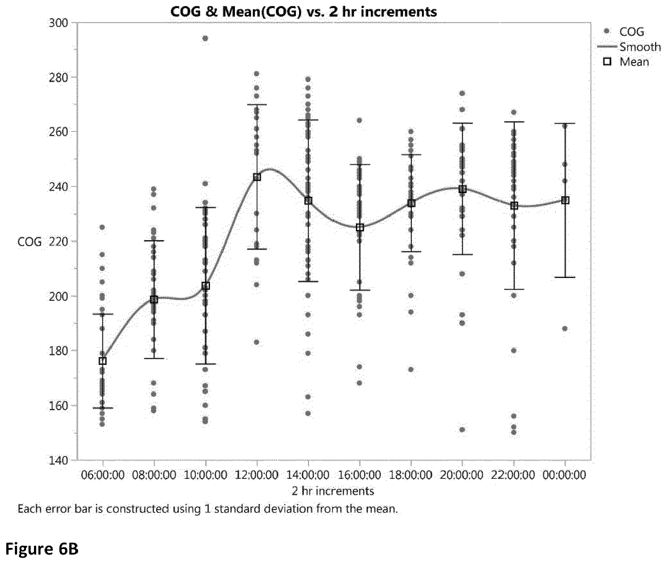

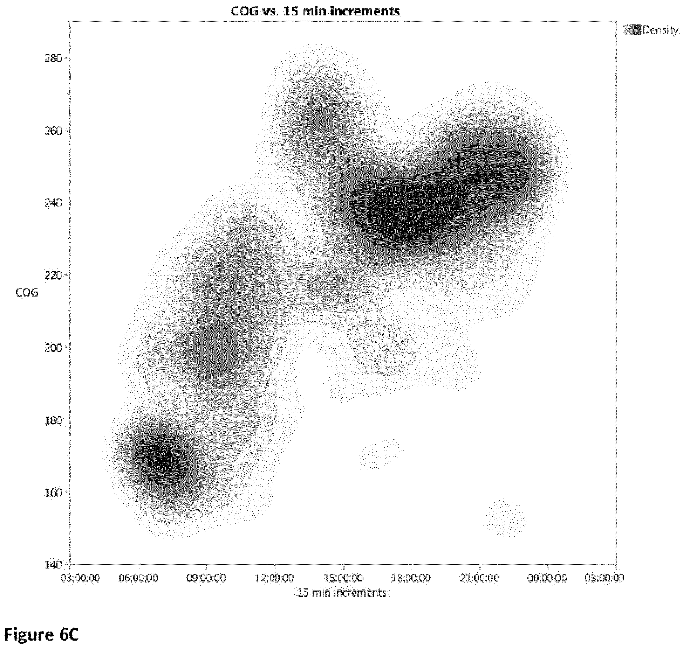

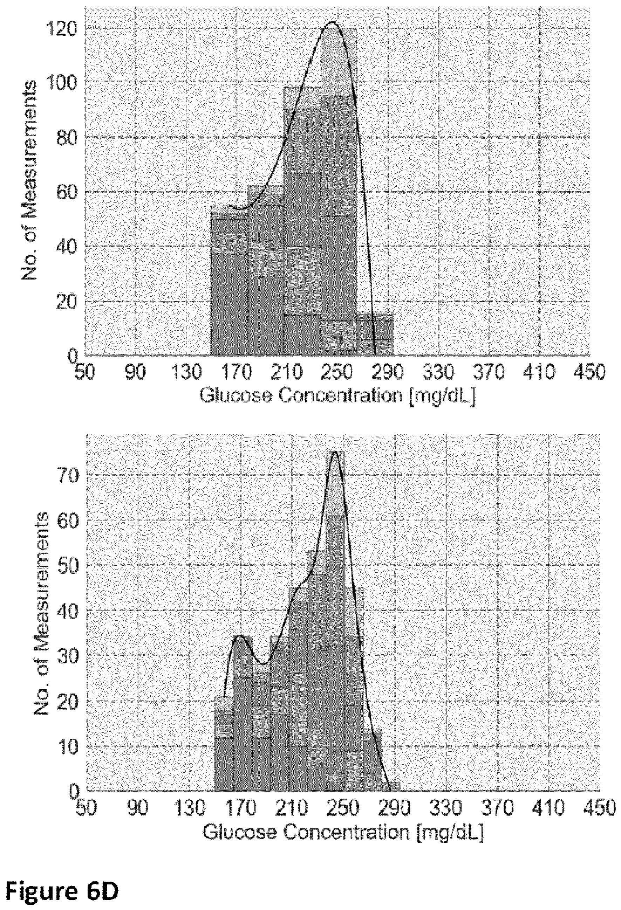

Beyond their metabolic variability, different patients have different daily routines, eating habits, physical activity and medication schedules. The glucose histogram, as well as other visualizations of glycemic behavior, can help us understand how the glucose levels behave during the day to day. This allows us to focus on the most problematic times of the day and target them specifically with behavioral and pharmacological interventions. For example, Figure 6A shows the distribution of glucose levels for a single patient during the 24-hour daily cycle, encompassing 350 measurements over 2 months. It can be clearly seen that the glycemic control is best in the morning, and as the day advances, the glucose histogram becomes more and more skewed. The scatter plot in Figure 6B shows the same data in a different way: as the morning progresses the glucose levels increase up to a peak around lunch time, with a continuation of these high levels throughout the remainder of the day. The large dispersion of the measures is also noteworthy, given that glycemic variability has been shown to be an independent risk factor for diabetes complications and a target for diabetes care. Figure 6C shows a density plot of the same dataset, binned into 15 minute slots, in what some may find is a more visually intuitive way. This plot focuses us into four clear clusters of glucose levels: A, early morning, showing fasting glucose levels of about 170 mg/dL; B, morning, where glucose levels increase steadily after breakfast; C, a high peak after lunch time; and finally D, continually high levels from afternoon through the night. Finally, figure 6D shows a histogram with the different times of day color coded, allowing us to get a rough sense of the dispersion of values during the day.

During Applicant's analysis of the data collected in its clinical trials, Applicant discovered that presenting the data as a glucose histogram would be valuable. Although some continuous glucose monitoring devices can show a histogram of some of their data, these cannot be readily manipulated or analyzed. The glucose histogram is a powerful tool for the characterization and diagnosis of diabetic patients and their management. The glucose histogram is also device agnostic, and thus it is applicable to any scenario where personal glucose data is continuously recorded.

The number of diabetic patients is very large and continues to grow. In order to improve their health and reduce complications, it is important to obtain as good a glycemic control as possible. If this is done using current treatment paradigms, it would be an intensive effort that would require enormous economic investment in healthcare which is almost certainly impossible to procure. Thus, in order to meet this need, certain embodiments of the invention move the center of diabetes care further towards the patient. It has already moved, for most patients, from specialists to primary care. Even for those that do go to specialists, their long term care is delivered mostly by primary care physicians. In some countries it is moving further to nurse practitioners. Using certain embodiments of the invention, diabetes care is a patient driven effort where specialists, primary care physicians and nurse practitioners become less central in the day to day management of diabetes and patients gain more control of their own health. There are three necessary conditions for this to happen, all of which are within reach with current technologies: combination of standard face to face care encounters with telemedicine follow up; digital collection of frequent glucose measurements in a format amenable to analysis and sharing; and use of patient level software tools to analyze and interpret the data. Certain embodiments of the invention use the glucose histogram to convert data into information, and extract from the information clinically actionable insights - both for healthcare personnel and patients.

Like many other variables and measures in modern medicine, the HBA1C threshold for diabetes diagnosis was developed from populations with specific ethnic backgrounds and then extrapolated to the whole world, which is known to be problematic. The glucose histogram can be of great use for the accurate diagnosis of patients with diabetes, prediabetes, or suspected diabetes, especially in the populations where the 6.5% HBA1C threshold has sensitivity problems. Beyond diagnosis, Figure 3 highlights how problematic it is to use the HBA1C as the main indicator of glycemic control in diabetic patients. Similar HBA1C values can be obtained from very different underlying glycemic behaviors that require different approaches for optimal control. A low value, which would be a sign of adequate control to most clinicians, can actually mask a significant derangement of the glucose metabolism. This strengthens the argument that the HBA1C is only a very rough estimate of our patients' state and it is questionable as a reliable way to compare among patients. The glucose histogram is a much more powerful indicator of the glycemic pathology of our patients and their disease state, containing personalized information that can be directly translated into individualization of treatment. The glucose histogram does this in a visual and intuitive manner that is easy to comprehend by physicians and patients alike. Independently of the benefit obtained by the physician for devising treatments, the glucose histogram provides ample space for better communicating to the patients the specific areas that need better control, as opposed to general recommendations. This type of educational and communication interventions has been shown to reduce mortality among diabetic patients.

Diabetic patients are heterogeneous, comprising a spectrum of pathophysiologic configurations. Many patients require multiple agents to achieve appropriate glycemic control, which have different mechanisms of action, secondary and idiosyncratic effects and interactions with other medications. It stands to reason then that these patients should benefit from therapy tailored to their specific diabetes subtype and response to treatment.

A method that includes following up with our patients in a more granular way is a prerequisite to perform this personalization. In this context, the different histogram shapes and their change in response to treatment greatly help one to understand the underlying problems of unique patients. In certain embodiments, the invention also correlates them with other patient information such as medications used and timing, insulin use patterns and food intake patterns, so that analysis of the histogram allows for a powerful, personalized approach to glycemic control. This is in stark contrast with current treatment recommendations that look at the HBA1C, propose general lifestyle changes and prescribe medications according to a standard flowchart or table. The dynamic representation of a patient's glycemic behavior analyzed over time, as shown in figure 4, is a further example of how these tools can help a clinician devise specific interventions, reinforcing a good strategy or modifying an inadequate one. In certain embodiments of the invention, the time frame of analysis is much faster than is common with HBA1C since dynamics can be monitored as soon as every two weeks. This allows clinicians to provide relevant, real time feedback and adjust the pharmacologic intervention as the patient changes into a new equilibrium.

Instead of general behavioral changes and adding medications according to standard algorithms while monitoring the HBA1C, the invention, according to certain embodiments, targets interventions towards changing the glucose histogram. For any given patient, therapeutic interventions should target regaining glycemic homeostasis and normalizing the glycemic pattern. In certain embodiments of the invention this approach has several advantages, among them: being able to monitor the response to treatment more dynamically than with HBA1C; being able to follow up the actual glucose values and not just HBA1C, accounting for hypo- and hyper-glycemia; and being able to assess measures of glycemic variability, an important yet underappreciated pathophysiological characteristic of diabetes which independently confers risk, and all this in a personalized, patient-specific manner.

The invention, in certain embodiments, moves from HBA1C and standard algorithms to methods that are information rich, harnessing the entire extent of glycemic data available at the personal level to provide individualized recommendations and care. This approach is highly amenable for implementation in informatics-assisted, patient-centered and patient-led care. In certain embodiments, this provides truly personalized care by intervening in the specific glycemic derangements of unique patients in their unique contexts.

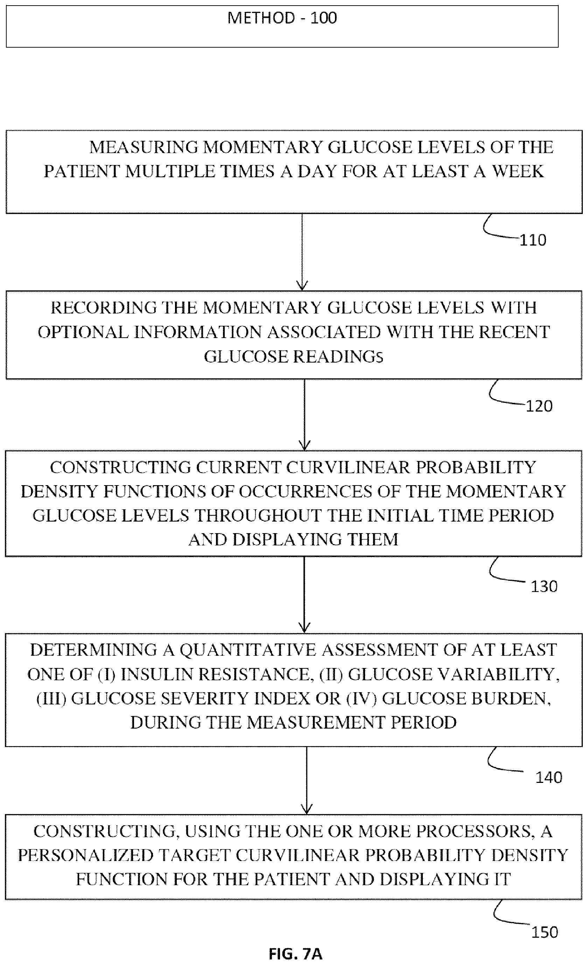

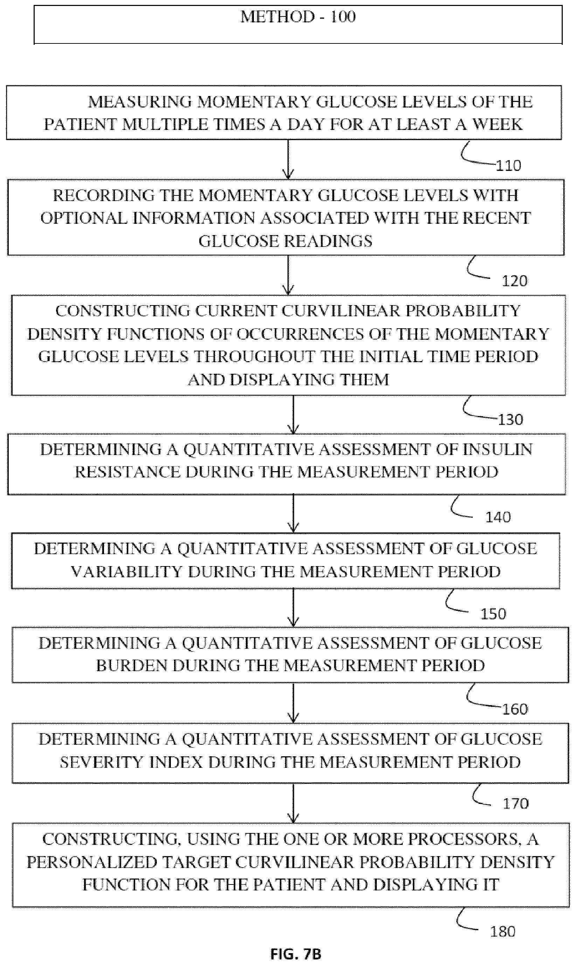

Accordingly, as shown in Fig. 7, one embodiment of the invention is a method 100 of for controlling blood glucose of a patient, for example a patient having diabetes, by using personalized histograms. The method 100 includes a step 110 of measuring, using a non-invasive measuring device 10 or in other embodiments using an invasive glucose measuring device 10, momentary glucose levels of the patient at multiple times a day during an initial time period, the initial time period being at least a week (or in other embodiments a month or two months or three months or at least a month or at least two months or at least three months). In some embodiments the measuring is continuous and in other embodiments the measuring is discrete measurements taken at discrete times. One non-limiting example of the non-invasive device is the TensorTip Combo Glucometer made by Cnoga Medical Ltd, Caesarea, Israel. The invasive unit of the CoG (Combo Glucometer) measures capillary blood glucose levels in the range of 40-440 mg/dL (2.2-24.2 mmol/L) obtained from lancing a fingertip. This add-on invasive module is identical to the Okmeter match device K090609 (OK Biotech Co, Ltd., Hsinchu City, Taiwan). The noninvasive unit is based on the TensorTip optical technology developed by Cnoga. In some embodiments, the measuring is performed at least a first time before meals and at least a second time after meals.

Method 110 may include a further step 120 of recording the momentary glucose levels in a memory of the device 10 or a memory in communication with the device. The recording, in some embodiments, includes optional information associated with the recent temporal glucose reading. The optional information includes one or more of the following information: date, time, time of recent food eating, physical activity, diabetes treatment, body temperature, blood pressure, pulse, and drugs in general or drugs (including specialized drugs) associated with the recent temporal glucose reading. For example, the date and time of the momentary glucose reading is recorded in some embodiments. In some embodiments, information about current diabetes treatment, physical activity (i.e. exercise) and/or drugs associated with the recent temporal glucose reading is/are recorded in conjunction with (or without) the date and/or time of the reading. Information about whether the time of the recoding is before or after meals is also recorded in some embodiments. In some embodiments, other bioparameters such as body temperature, blood pressure and/or pulse are also recorded.

In certain embodiments, the time zone is inserted into the histogram. This is depicted in Fig. 6A, Fig. 6B, Fig. 6C and Fig. 6D. Figure 6A represents glucose histograms of a type 2 diabetes patient divided by times of the day, encompassing 350 measurements over two months, in accordance with one embodiment of the invention. The morning histogram (06:00 to 12:00) is on top, the afternoon histogram (12:00 to 18:00) in the middle and the evening histogram (18:00 to the next morning) at the bottom. Although in Fig. 6A colors were used to differentiate the different time zone histogram data, other visual distinctions or designations may also be used. Figure 6B depicts the same data as 6A, which has been binned into 2 hour slots. In this non-limiting example, the blue dots represent the individual measures, the squares are the mean glucose value for that bin, and the red line represents the trend line. The error bars represent

$\pm 1$ SD from the mean. Figure 6C shows the same data as 6A, with time binned into 15 minute slots and the glucose levels binned into 8 steps. The darker areas represent higher density of measurements in that region. Figure 6D shows the same data as 6A. The Glucose distribution of the type 2 diabetes patient is presented by stacked histograms, color coded by different hours during the day. The lower histogram shows the same data with the histogram created using smaller bins.

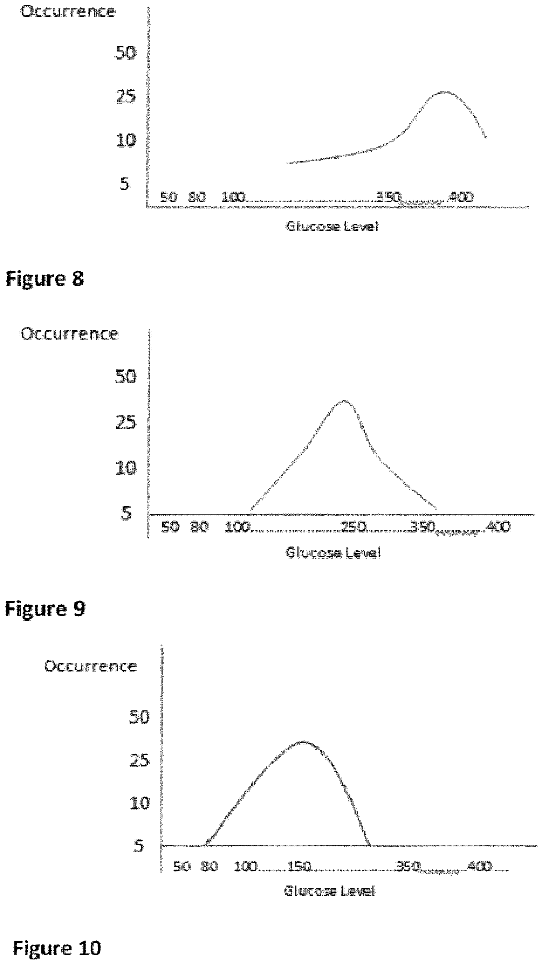

Method 100 may also include a step 130 of constructing, using one or more processors of the device or in communication with the device, current curvilinear probability density functions of occurrences of the momentary glucose levels throughout the initial time period. An example of the results of such constructing is shown in Fig. 8. Step 130 may also include displaying (and/or printing and/or electronically transmitting) the curvilinear probably density functions on a screen for viewing by a patient and/or caregiver and/or medical professional, as shown in Fig. 8. All software and hardware needed to implement the invention is included in a processing unit that is in communication with the device that performs the measuring of step 110 or else is in communication with such device 10.

The curvilinear probability density functions may be constructed directly from the momentary glucose levels recorded in step 120 or they may be constructed from glucose histograms that were constructed from the momentary glucose levels recorded in step 120 by one or more processors. In some embodiments, the glucose histograms or the curvilinear probability density function is computed to represent the momentary glucose levels during the time between $T_0$ to $T_1$ in the period of time P. For example, the temporal histogram is computed to represent the glucose level between 13:00 to 14:00 (1:00 p.m. to 2:00 p.m.) each day during the month of February (P - Period). The displaying is useful for the patient or health practitioner to see what is going on graphically regarding the diabetes control. The power of graphical tools for glucose data visualization and analysis such as the ones we present is further demonstrated when looking at the daily glucose patterns of the patients in Applicant's study, as in Figure 6. In some embodiments, intuitive graphs are used to show a patient's actual glycemic control daily as the basis for glycemic control. In contrast, using state of the art recommendations, the only personal measure would be the patient's HBA1C. The method 100 uses new forms of visualization and once the actual momentary glucose data is recorded, it is unnecessary in some embodiments to even make use of the HBA1C. In the case shown in Figure 6, for example, the HBA1C is not even mentioned, yet that information does not seem necessary to assess that patient's condition and propose effective interventions (even more so if the data is coupled with information regarding the patient's habits and therapeutic regime).

Furthermore, this approach to diabetes control need not be restricted to insulin users. A type 2 diabetic not treated with insulin might nonetheless borrow a non-invasive monitor from his health provider and do intensive self-monitoring in a convenient and painless way for a couple of weeks. The data obtained could then be used to guide specific recommendations in medications and habits, and the process repeated a few months later.

One should not underestimate the major contribution of the cognitive processes, habits and perceptions of the patients on their health outcomes. Like most other chronic diseases, diabetes care does not end but just begins with the professional medical recommendations. The difficulty implementing those recommendations by our patients into their lives is a major barrier which is as complex and multifactorial as the diseases themselves, and perhaps even more individual. Thus, presenting the data in the patient's actual context, engaging the patient with analyses that he can understand and obtain actionable insights and providing solutions that he can integrate as smoothly as possible in his daily routine can be no less effective than a change of medication, and perhaps even more so.

In accordance with some embodiments, method 100 may further include incorporating the optional information together with the curvilinear probability density function(s) so that one or more processors can integrate the data together.

Method 100 also includes, in some embodiments, a step 140 or steps 140 and 150 or steps 140, 150 and 160 of determining, using the one or more processors, from the curvilinear probability density functions and the optional information, a quantitative assessment of at least one of (or at least two of or all four of): (i) insulin resistance during the measurement period, (ii) glucose variability during the measurement period, (iii) glucose burden or (iv) glucose severity index during the measurement period, is determined.

In one version method 100 includes each of steps 140, 150 and 160. For example, method 100 may include a step 140 of determining, using the one or more processors, from the curvilinear probability density functions and the optional information, a quantitative assessment of insulin resistance (IR) during the measurement period from the curvilinear probability density functions and the optional information. In one non-limiting example, insulin resistance (IR) is determined in some embodiments by for example by collecting at least 5 times per day optimal is 8 times per day glucose readings (e.g. wakening, before breakfast, after breakfast, before lunch, after lunch, before dinner, after diner and before bed time) for at least a week assuming for this example the period of time P is two weeks and if used without any treatment (i.e. the optional information) one may provide rough estimation in the following form

$$\beta = \left[1 + \frac{N_{gs}}{N}\right] \cdot \left[1 + \frac{1}{N} \sum_{k=1}^{N_{gs}} \frac{g_k - g_s}{g_k}\right] \quad if \quad g_k \geq g_s$$

where in this example $g_s = 150 \frac{mg}{dL}$ . In some other embodiment $g_s$ is set to a different value. $Ng_s$ represents the total measurements greater than $g_s$ and N the total glucose readings (measurements) collected during the period of time P. if non such measurements acquired during the period of measurement one may conclude that $\beta = 1$ which means that IR is normal during the measurement period P. However, if for example 65 out of 128 total glucose readings were above $g_s$ and $1 + \frac{1}{N} \sum_{k=1}^{N_{gs}} \frac{g_k - g_s}{g_k}$ resulted in 1.144, then $\beta = \left(1 + \frac{65}{128}\right) \cdot 1.144 = 1.5 \cdot$ 1.144 = 1.72 which roughly indicates a suspicion of an above-normal IR. Yet, the above formula could be improving once the additional information regarding meals, physical activity and drug treatments are provided.

Method 100 also includes, in some embodiments, a step 150 of determining, using the one or more processors, a quantitative assessment of glucose variability (GV) during the measurement period from the curvilinear probability density functions and the optional information. According to one non-limiting example of how glucose variability is defined, the one or more processors determine the average rate of change of the glucose level during the measurement period and for example this may be determined by taking the total change in momentary glucose levels over the total time period. For example, if the patient's momentary glucose level changed (in mg/dL) ten times from 50 to 500 and vice versa (ending at 500) and then five times to 100 and vice versa (ending at 100) and then fifteen times from 100 to 400 and vice versa (ending at 400) and then 5 times to 200 and vice versa (ending at 200) during the recording period, that would add up to a total variability of (10*(500-50) + 5*(500-100) + 15*(400-100) +5(400-200))/35 = 342.85 mg/dL.

According to another non-limiting example of how glucose variability is defined, the one or more processors determine the gap between the lower and the higher glucose levels during period T. For example, GV is a selected gap representing the difference between two glucose levels $g_l$ and $g_k$ during period T where $T \leq P$ and P stands for the period of collecting the momentary glucose histogram readings. Typically, another glucose level, $g_i$, exists (and may be measured during T) and is between $g_l$ and $g_k$ i.e. $g_l < g_i < g_k$. In some cases, the selected gap is not the maximal gap during the time T; rather the selected gap is that gap that has the highest frequency of appearances among all gaps during time period T. In other cases, GV is the maximal gap during the time T.

In certain embodiments, determining the Glucose Severity Index (GSI) may provide a better insight than glucose variability (GV). An example of the computation of GSI is provided below. Accordingly, method 100 also includes in some embodiments a step of determining a quantitative assessment, using the one or more processors, the glucose severity index during the measurement period from the curvilinear probability density functions and the optional information.

Method 100 also includes, in some embodiments, a step 160 of determining, using the one or more processors, a quantitative assessment of glucose burden during the measurement period from the curvilinear probability density functions and the optional information. In one non-limiting example, the glucose burden is determined by determining the first moment of the curvilinear probability density function during the measurement period or directly from the histogram. Let f(g) be the glucose curvilinear probability density function or in some other embodiment the glucose occurrence histogram collected during a period of time, wherein "g" is the momentary glucose value along the x axis and the frequency of the momentary glucose value is along the y = f(g) axis. The glucose burden for example can be computed as the first moment resulting from f(g) and g as follows:

$$GB = A * \sum_{i=1}^{N} g_i * f(g_i)$$

where GB stands for "Glucose Burden" during the measured period of the glucose histogram, A is a positive constant that could be used as a normalization factor, and N is the total readings during the measurement period collected of the momentary glucose levels. This manner of determining the glucose burden is merely a non-limiting example of how to determine it. The glucose burden captures the average glucose level during the measurement period. Therefore, if the glucose burden is high over a sufficiently long period then damage to the patient's organs can occur.

Estimating GSI would be by using various simple equations in the following forms:

1.

$$\varphi = 1 + \frac{1}{N} \sum_{i=1}^{N} \left( \frac{|g_i - 100|}{\max(g_i, 100)} \right)^a$$

2.

$$\overline{GB} = 1 + \frac{|GB - 100|}{\max(GB, 100)}$$

3.

$$F_H = 2 - \frac{1}{T_p}$$

4.

$$F_T = 1 + \frac{2}{N} F$$

We consider without losing generality 100mg/dL as the normal glucose burden for people above 40 years of age. In other embodiments normal range may be considered a figure between 90mg/dL to 120mg/dL depending on the population group (age, healthy, diabetes type1, diabetes type2, ethnic, gender, etc.). $\varphi$ represents a weighted average glucose deviation from 100mg/dl. "a" is a positive real constant. "a" is used to manipulate $\varphi$ in a way one may wish to increase or reduce the $\varphi$ weight. $\varphi$ = 1 represents the best scenario and $\varphi$ = 2 represents the worst scenario ("best" and "worst" refer to how close the outcome is to 100mg/dL). $\overline{GB}$ represents a relative weighted distance from GB to 100mg/dl. $\overline{GB}$ = 1 represents the best scenario while $\overline{GB}$ = 2 represents the worst scenario. $F_H$ represents a weight resulting from the total local maxima in the density glucose histogram while Tp is the total local maxima. A local maximum is considered a local peak in the histogram wherein a local peak is greater than its left and right neighbours.

Density glucose histogram of a Gaussian shape with a single maximum represents the best scenario regardless of where the maximum occurrence occurs. For example, the maximum occurrence may occur at 90mg/dL or 300mg/dL and both cases represent Gaussian shape. Best scenario does not mean best in terms of glucose values; rather it means best in terms of the histogram shape. More peaks increase the value of $F_H$. The worst scenario is a straight line where there are no single global maxima, rather a continuous global maxima. $F_T$ represents a temporal frequency of the glucose readings during the measurement period. One may compute it using a Fast Fourier Transform (FFT) or by just counting the temporal peaks. The worst scenario occurs when F = N/2, i.e. data is intertwined N/2 times regardless of the gap. Thus, the worst scenario occurs where F = N/2 resulting in $F_T$ = 2. The best scenario occurs when F = 0. In that case $F_T$ = 1 regardless of the glucose burden level. In other words, F = 0 best scenario may occur where all glucose levels are at 300mg/dL or 90mg/dL as a straight temporal line.

GSI can be estimated by using a combination of the above formulas. For example, considering all scenarios:

○

$$GSI = F_H * \varphi * \overline{GB} * F_T$$

Additional scenarios include the following:

○

$$GSI = F_H * \varphi * F_T$$

○

$$GSI = F_H * \overline{GB} * F_T$$

$$\circ$$

$$GSI = F_H * \varphi$$

$$\circ$$

$$GSI = \varphi * \overline{GB} * F_T$$

GSI = 1 represents the best scenario under this test for Glucose Severity Index. However, when GSI increases, the glucose severity increases. Examples are shown in figures 1A, 1B and 1C.

For a better treatment the following path is considered:

1. Step A: consider the values of GB, GSI and the current histogram shape.
2. Step B: Verify that the new GSI is lower than the previous GSI and GB is improved versus the previous GB. Verify that the new density histogram shape matches or is sufficient close to the desired future target glucose histogram shape.

Other variations of the above formulas can be added. For example, one may wish to increase $\varphi$ weight for glucose level in range less than 70mg/dL. In that case use the constant a>0 such that whenever the glucose reading $g_i$ is lower than 70 choose "$\alpha$" to be less than one.

It should be noted that the Insulin Resistance (IR) referred to in step 140 during the measured period P may be estimated from at least one of (i) GSI components, (ii) the histogram or (iii) curvilinear probability density function. The estimation of insulin resistance (IR) may be improved by taking into consideration any or all of the optional information that may be available.

Figure 1A represents the shape of a curvilinear probability density function generated from a histogram of an actual healthy individual with a glucose burden of 92mg/dL and a total calculated GSI of 1.45 ($\varphi$=1.13, $\overline{GB}$=0.92, $F_T$=1.39, $F_H$=1). HGBA1C is estimated to be 4.8%. Figure 1B, in contrast, represents an actual severely unbalanced type 2 diabetic, with elevated glucose levels overall and a tri-modal distribution, with a glucose burden of 256 mg/dL and a total calculated GSI of 6.33 ($\varphi$=1.59, $\overline{GB}$=1.61, $F_T$=1.49, $F_H$=1.67). HGBA1C is estimated to be 10.5%. Figure 1C represents an actual labile type 1 diabetic, who suffers from several episodes of both hypo- and hyper-glycemia (29% of the readings are below 80 md/dL and 8% of the readings are above 200 mg/dL), with a glucose burden of 144 mg/dL and a total calculated GSI of 4.02 (<p=1.34, $\overline{GB}$=1.26, $F_T$=1.58, $F_H$=1.5). HGBA1C is estimated to be 6.6%.

Method 100 also includes, in some embodiments, a step 170 of determining, using the one or more processors, at least one of a diabetes type and a degree of severity of the patient's diabetes. Examples of a diabetes type include type I and type II. Examples of severity of the condition include light (where insulin resistance is not high and the pancreas produces insulin and which may only require diet and exercise), moderate (which may involve a high insulin resistance but the pancreas produces insulin and which may require drugs) and severe, meaning insulin dependent, where drugs are unworkable because the patient's pancreas does not produce insulin.

Method 100 also includes, in some embodiments, a step 180 of constructing, using the one or more processors, a personalized target curvilinear probability density function for the patient based on the current curvilinear probability density functions, the insulin resistance (IR), the glucose severity index (GSI), the glucose burden (GB) or based on the quantitative assessment of at least one of (or at least two of): (i) the insulin resistance, (ii) the glucose variability, the glucose severity index or (iv) the glucose burden, and the optional information. The IR, GV, GSI and GB are current momentary levels of these parameters which are called biomarkers (or simply markers). In the step 180 of constructing the personalized target curvilinear probability density function for the patient, the one or more processors may also consider the at least one of a diabetes type and the degree of severity of the diabetes. The constructing of the personalized target curvilinear probability density function for the patient performed in step 180 involves constructing a future personal target for the patient.

In some embodiments method 100 may also include a step 180 of displaying (and/or printing and/or electronically transmitting) the personalized target curvilinear probability density function on the screen for viewing by the patient, the caregiver and/or the medical professional. An example of such a personalized target curvilinear probability density function for the patient is shown in Fig. 9.

The one or more processors 14 may be part of the processing unit 14a that may also include the memory 12 and program instructions 16. All program instructions 16 or software 16 necessary to implement the steps that utilize or involve the one or more processors 14 are included in the processing unit 14a or are downloadable or accessible by the processing unit 14a.

A suggested personal treatment is outputted in some embodiments based on the analysis of the one or more processors. The suggested treatment is at least one of the following: diet (sometimes called "food and diet"), rest and physical activity, insulin and other drugs.

In some embodiments, method 100 also further comprises verifying a success of the personal treatment by at least one of (i) verifying improvement of one or more of the IR and the GB compared to a respective IR and GB of a previous period and (ii) verifying reduction of a level of GSI compared to a level of GSI in a previous period, and by verifying a match of a new curvilinear probability density function constructed based on momentary glucose levels measured after the personal treatment with the target curvilinear probability density function. The match is determined based on pre-determined quantitative criteria.

In some embodiments, the personalized target curvilinear probability density function reflects a severity of diabetes that is less severe than the severity reflected by the shape of the current curvilinear probability density functions. This is consistent with its purpose, which is to design an improved diabetes control condition. For example, during the initial time period shown in Fig. 8, the condition was severe (uncontrolled type 2) whereas the personalized target curvilinear probability density function shown in Fig. 9 for the same patient reflects a less severe condition of diabetes.

Accordingly, in some embodiments, the personalized target curvilinear probability density function may be designed to improve the patient's control of diabetes in more than a single stage. For example, Fig. 9 reflects a first stage of personal improvement designed as a personalized targeted curvilinear density function and Fig. 10 reflects a second stage of personal improvement designed as a personalized targeted curvilinear density function. In Fig. 10, the second step of the controlling process is to continue shifting the histogram to the left while avoiding hypo stage and keeping the Gaussian Bell shape. After 3-month of additional controlling the max histogram is achieved at about 150mg/dL resulting in average glucose level of 150mg/dL representing an estimated 6.8% A1C.

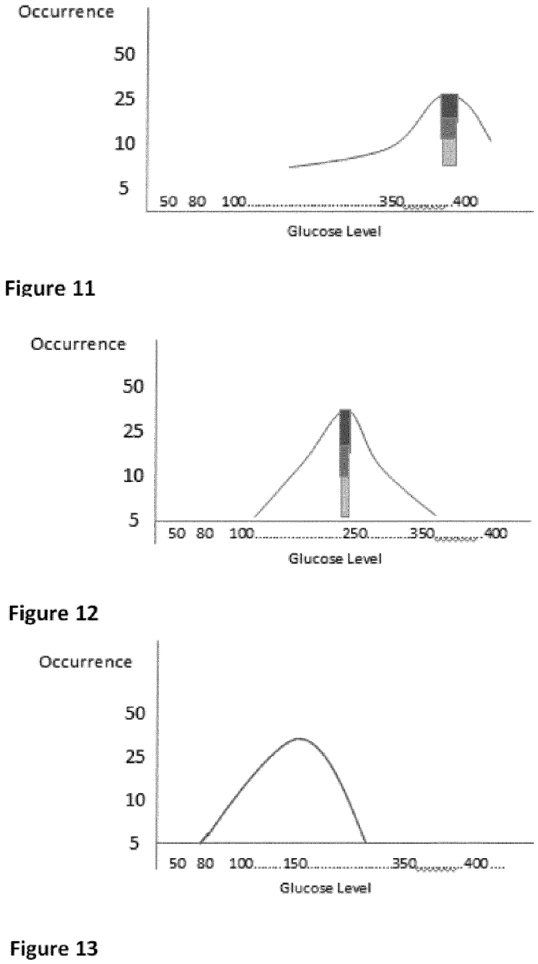

Figures 11-13 show a similar concept as in Figs. 8-10 except that the time zone within the day (of the momentary glucose readings) is depicted in the graphs and taken into consideration in some embodiments. For example, Fig. 11 depicts an initial 3-month histogram of an uncontrolled individual with estimated 9.3% A1C. Most of the higher glucose occurs at the zone time set above. Fig. 12 shows that in this embodiment the first target or first step in the process of controlling the diabetes is to shift the histogram to the left so that the max histogram location (i.e. about 400mg/dL) will be shifted to a mid-value with a normal Gaussian shape. After 3 months of control the max histogram is achieved at about 250mg/dL with a fairly normal Gaussian shape. The treatment was focused on the zone time set above. Figure 13 shows that in this embodiment the second step of the controlling process is to continue shifting the histogram to the left while avoiding hypo stage and keeping the Gaussian Bell shape. After 3-month of additional controlling in this second stage the max histogram is achieved at about 150mg/dL resulting in average glucose level of 150mg/dL representing an estimated 6.8% A1C.

In some embodiments, there are more than two stages of personal improvement, wherein each stage utilizes a personalized targeted curvilinear density function.

The one or more processors, in certain embodiments, also determine quantitatively an extent to which the target curvilinear shape of the patient's curvilinear probability density function was realized during the second time period. For example, Fig. 9 is more Gaussian-like shape than Fig. 8 is and Fig. 10 is more Gaussian-like in shape than Fig. 9. The degree that a graph is Gaussian in shape can be quantified and the results of the treatment monitored quantitatively in this manner, in some embodiments. Accordingly, in some embodiments, the target curvilinear probability density function is a Gaussian-like shape.

In some embodiments of method 100, the personalized target curvilinear probability density function is used to suggest a treatment, wherein the suggested treatment is at least one of the following: diet, physical activity, insulin and drugs. In some embodiments, method 100 includes a step of monitoring a response to the treatment including (i) monitoring, and recording a quantitative extent of, a change over time in the shape of the curvilinear probability density function in a direction of being more Gaussian (which change in shape can occur in a first (or second) stage of personal improvement in the treatment approach); and (ii) monitoring, and recording a quantitative extent of, a shift of the curvilinear probability density function reflecting a lowering of a glucose level at which one or more peaks appear in the curvilinear probability density function (which change in shape can occur in the second (or first) stage of personal improvement in the treatment approach).

In some embodiments, method 100 further comprises outputting at least one of (i) a type of diabetes, an existence of hyperglycemia, an existence of hypoglycemia, and a degree of severity of the diabetes and (ii) a tentative diagnostic suggestion. In other embodiments, method 100 comprises outputting at least one of (i) a type of diabetes, an existence of hyperglycemia, an existence of hypoglycemia, and a degree of severity of the diabetes, (ii) a tentative diagnostic suggestion and (iii) a tentative treatment suggestion.

In some embodiments, the one or more processors compare the target histograms of different periods of time.

In another embodiment of the method 100, instead of the method including each of steps 140, 150 and 160, the method 100 comprises a step of determining, using the one or more processors, a quantitative assessment of at least one of (or in other embodiments at least two of or at least three of or all four) (i) insulin resistance , (ii) glucose variability, (iii) glucose severity index , (iii) glucose burden, all of which are from the glucose readings of the patient during the measurement period.

For example, the invention in one embodiment is a method of control of diabetes in a patient, comprising measuring, continuously or at discrete times, using a non-invasive or invasive glucose measuring device, momentary glucose levels of the patient at multiple times a day during a time period, the initial time period being at least a week; recording the momentary glucose levels in a memory in communication with the device, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading or drugs in general; constructing, using one or more processors in communication with the device, current curvilinear probability density functions of occurrences of the momentary glucose levels throughout specific hours of the day during the time period and displaying the curvilinear probability density functions on a screen; determining, using one or more processors, a quantitative assessment of at least one of (i) insulin resistance during the measurement period from the glucose readings of the patient, (ii) glucose variability during the measurement period from the glucose readings of the patient, (iii) glucose burden during the measurement period from the glucose readings of the patient; determining, using the one or more processors, at least one of a diabetes type and a degree of severity of the patient's diabetes; and constructing, using the one or more processors, a personalized target curvilinear probability density function for the patient based on the current curvilinear probability density functions and optionally based also on the at least one of (i) the insulin resistance, (ii) the glucose variability, (iii) the glucose severity index or (iv) glucose burden, and the optional information. The personalized target curvilinear probability density function may be displayed on the screen.

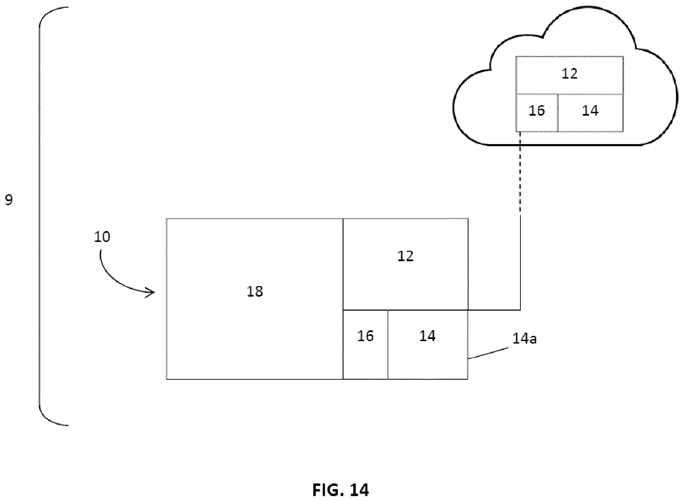

As shown in Fig. 14, one embodiment of the invention is a system 9 for controlling blood glucose of a patient by using personalized histograms. The various non-limiting embodiments of the histograms, the current and target curvilinear probability density functions, the biomarkers and their quantitative assessments and the optional information, etc. used by the one or more processors of system 9 has already been discussed above including in the context of method 100.

For example, system 9 comprises a non-invasive or invasive glucose measuring device 10 configured to either continuously or at discrete times measure glucose levels of the patient including at multiple times a day during an initial time period, the initial time period being at least a week.

System 9 also includes a storage memory 12 for recording the momentary glucose levels, the memory in communication with the device 10, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading. Memory 12 may be within device 10 or may be external to device 10 (for example but not necessarily in cyberspace).

System 9 also includes one or more processors 14 (in some cases of a processing unit 14a) in device 10 and in communication with memory 12 or external to device 10 and in communication with the memory 12 and the glucose measuring device 10. The one or more processors 14 are for constructing, current curvilinear probability density functions of occurrences of the momentary glucose levels throughout the initial time period and displaying the curvilinear probability density functions on a screen 18, for example a screen 18 of device 10, as seen in Fig. 14. If the one or more processors are external to device 10 (an alternative also shown in Fig. 14) then the glucose data is transmitted either via wired connection or wirelessly to an external memory 12 to be used by the one or more processors for the glycemic control. The constructed current curvilinear probability density functions are displayed visually on a screen 18 and/or printed for visual review and/or electronically transmitted to the patient and/or to medical professionals and/or to health care workers or assistants of the patient. Program instructions 16 or software are stored on memory 12 and accessible to the one or more processors 14.

In certain embodiments, the one or more processors 14 are configured to determine a quantitative assessment of at least one of the following current momentary markers during the measurement period from the momentary glucose levels recorded and the optional information: (i) Insulin Resistance (IR), (ii) Glucose Variability (GV), (iii) Glucose Severity Index (GSI) or (iv) Glucose Burden (GB).

The one or more processors 14, in some embodiments, are configured to also construct a personalized target curvilinear probability density function for the patient based on the current curvilinear probability density functions. The constructing of the personalized target curvilinear probability density function for the patient is based on the quantitative assessment of the one or more biomarkers in some embodiments. In some embodiments, the constructed target functions are displayed visually on a screen 18 and/or printed for visual review and/or electronically transmitted to the patient and/or to medical professionals and/or to health care workers or assistants of the patient.

In some embodiments, the one or more processors are configured to verify a success of a personal treatment by at least one of (i) verifying improvement of one or more of the IR, GV and the GB compared to a respective IR, GV and GB of a previous period and (ii) verifying reduction of a level of GSI compared to a level of GSI in a previous period, and by verifying a match of a new curvilinear probability density function constructed based on momentary glucose levels measured after the personal treatment with the target curvilinear probability density function.

In some embodiments, the one or more processors are further configured to determine the quantitative assessment of at least one of the following current momentary markers during the measurement period from the momentary glucose levels recorded and the optional information: (i) insulin resistance (IR), (ii) glucose severity index (GSI), (iii) glucose burden (GB) and (iv) glucose variability and to construct the personalized target curvilinear probability density function for the patient, the target curvilinear probability density function configured to improve at least one of (i) the IR, (ii) the GSI, (iii) the GB and (iv) the GV of the patient.

In some embodiments, the one or more processors are configured to construct the curvilinear probability density functions from glucose histograms.

In some embodiments, the curvilinear probability density functions are the glucose histograms.

In some embodiments, the one or more processors are configured to construct glucose histograms that represent the occurrences of the momentary glucose levels throughout only certain hours of day over a time period, time period spanning all or part of the initial time period.

In some embodiments, the suggested diabetes treatment is at least one of the following: diet, rest, physical activity, insulin and drugs.

In some embodiments, the personalized target curvilinear probability density function reflects a severity of diabetes that is less severe than the severity reflected by the shape of the current curvilinear probability density functions during the initial time period for the same patient.

In some embodiments, the personalized target curvilinear probability density function is configured to improve at least one of the following quantitative assessments: GV, GSI, IR and GB of the patient, wherein improving GSI means reducing a frequency of glucose above a predefined amount level as well as improving the glucose burden and shifting the target histogram to have a single peak closer to a normal glucose value, improving GB means reducing GB level for instances of hyperglycemia and increasing GB level for instances of hypoglycemia and improving IR means improving absorption of glucose in the cells of the patient's body.

In some embodiments, the personalized target curvilinear probability density function reflects a first stage of personal improvement that precedes a second stage and any further stages of personal improvement designed by a personalized targeted curvilinear density function.

In some embodiments, the curvilinear probability density functions include the optional information as a look up table or as an additional axis. In some embodiments, the optional information includes the time of recent food eating. In some embodiments, the optional information includes the physical activity. In some embodiments, the optional information includes the diabetes treatment.

In some embodiments, the one or more processors are configured to determine a quantitative measure of an extent to which the target curvilinear shape of the patient's curvilinear probability density function was realized during the second time period.

In some embodiments, the target curvilinear probability density function is a Gaussian-like shape.

In some embodiments, the one or more processors are configured to monitor a response to a diabetes treatment including

(i) monitoring, and recording a quantitative extent of, a change over time in the shape of the curvilinear probability density function in a direction of being more Gaussian; and
(ii) monitoring, and recording a quantitative extent of, a shift of the curvilinear probability density function reflecting a lowering of a glucose level at which one or more peaks appear in the curvilinear probability density function.

In some embodiments, the one or more processors are configured to generate an output of at least one of

(i) a type of diabetes, an existence of hyperglycemia, an existence of hypoglycemia, and a degree of severity of the diabetes;
(ii) a tentative diagnostic suggestion; and
(iii) a tentative treatment suggestion.

In some embodiments, the multiple times of each day in the initial time period includes at least a first time before a meal and a second time after a meal.

The invention can be adapted to other bio markers such as blood pressure, cardiac output, stroke volume, cardiac output and stroke volume variation.

As used in this patent application, the phrase structure "at least one of (i) A and (ii) B" shall be understood to mean the same as "at least one of (i) A or (ii) B". Both phrase structures include "A", "B" and any combination of "A" and "B". The same applies to phrase structures with longer lists, such as "at least one of (i) A, (ii) B and (iii) C" and "at least one of (i) A, (ii) B or (iii) C".

Memory in communication with the device for measuring the glucose levels includes a scenario in which the memory is internal to the device and includes a scenario of external memory.

While the invention has been described with respect to a limited number of embodiments, it will be appreciated that many variations, modifications and other applications of the invention may be made. Therefore, the claimed invention as recited in the claims that follow is not limited to the embodiments described herein.

**Claims**

1. A method of control of diabetes in a patient, comprising:

   measuring, using a non-invasive or invasive glucose measuring device, momentary glucose levels of the patient at multiple times a day during an initial time period either continuously or at discrete times, the initial time period being at least a week;
   recording the momentary glucose levels in a memory in communication with the device, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading;
   constructing, using one or more processors in communication with the device, current curvilinear probability density functions of occurrences of the momentary glucose levels throughout the initial time period and displaying the curvilinear probability density functions on a screen;
   determining, using one or more processors, a quantitative assessment of at least one of the following current momentary markers during the measurement period from the momentary glucose levels recorded and the optional information: (i) Insulin Resistance (IR), (ii) Glucose Variability (GV), (iii) Glucose Severity Index (GSI) or (iv) Glucose Burden (GB), and
   constructing, using the one or more processors, a personalized target curvilinear probability density function for the patient based on the current curvilinear probability density functions.

2. A system for controlling blood glucose of a patient by using personalized histograms, comprising:

   a non-invasive or invasive glucose measuring device configured to either continuously or at discrete times measure glucose levels of the patient including at multiple times a day during an initial time period, the initial time period being at least a week;
   a storage memory for recording the momentary glucose levels, the memory in communication with the device, the recording including optional information associated with a recent temporal glucose reading wherein the optional information including one or more of the following information: date, time, time of recent food eating, recent physical activity, diabetes treatment, body temperature, blood pressure, pulse, and drugs associated with the recent temporal glucose reading;
   one or more processors either in the glucose measuring device or external to the device and in communication with the glucose measuring device and the memory, for constructing, current curvilinear probability density functions of occurrences of the momentary glucose levels throughout the initial time period and displaying the curvilinear probability density functions on a screen;
   the one or more processors configured to determine a quantitative assessment of at least one of the following current momentary markers during the measurement period from the momentary glucose levels recorded and the optional information: (i) Insulin Resistance (IR), (ii) Glucose Variability (GV), (iii) Glucose Severity Index (GSI) or (iv) Glucose Burden (GB), and
   the one or more processors configured to also construct a personalized target curvilinear probability density function for the patient based on the current curvilinear probability density functions.

3. The method of claim 1 or the system of claim 2, wherein the one or more processors are configured to verify a success of a personal treatment by at least one of (i) verifying improvement of one or more of the IR, GV and the GB compared to a respective IR, GV and GB of a previous period and (ii) verifying reduction of a level of GSI compared to a level of GSI in a previous period, and by verifying a match of a new curvilinear probability density function constructed based on momentary glucose levels measured after the personal treatment with the target curvilinear probability density function.

4. The method of claim 1 or the system of claim 2, wherein the one or more processors are configured to determine the quantitative assessment of the at least one of the following current momentary markers during the measurement period from the momentary glucose levels recorded and the optional information: (i) insulin resistance (IR), (ii) glucose severity index (GSI), (iii) glucose burden (GB) and (iv) glucose variability and constructing the personalized target curvilinear probability density function for the patient, the target curvilinear probability density function configured to improve at least one of (i) the IR, (ii) the GSI, (iii) the GB and (iv) the GV of the patient.

5. The method of claim 1 or the system of claim 2, wherein the one or more processors are configured to construct the curvilinear probability density functions from glucose histograms.

6. The method of claim 1 or the system of claim 2, wherein the curvilinear probability density functions are the glucose histograms.

7. The method of claim 1 or the system of claim 2, wherein the one or more processors are configured to construct glucose histograms that represent the occurrences of the momentary glucose levels throughout only certain hours of day over a time period, time period spanning all or part of the initial time period.

8. The method of claim 1 or the system of claim 2, wherein the personalized target curvilinear probability density function reflects a severity of diabetes that is less severe than the severity reflected by the shape of the current curvilinear probability density functions during the initial time period for the same patient.

9. The method of claim 1 or the system of claim 2, wherein the personalized target curvilinear probability density function is configured to improve at least one of the following quantitative assessments: GV, GSI, IR and GB of the patient, wherein improving GSI means reducing a frequency of glucose above a predefined amount level as well as improving the glucose burden and shifting the target histogram to have a single peak closer to a normal glucose value, improving GB means reducing GB level for instances of hyperglycemia and increasing GB level for instances of hypoglycemia and improving IR means improving absorption of glucose in the cells of the patient's body.

10. The method of claim 1 or the system of claim 2, wherein the curvilinear probability density functions include the optional information as a look up table or as an additional axis and wherein.
the optional information includes at least one of (i) the time of recent food eating, (ii) the physical activity and (iii) the diabetes treatment.

11. The method of claim 1 or the system of claim 2, wherein the one or more processors are configured to determine a quantitative measure of an extent to which the target curvilinear shape of the patient's curvilinear probability density function was realized during the second time period.

12. The method of claim 1 or the system of claim 2, wherein the target curvilinear probability density function is a Gaussian-like shape.

13. The method of claim 1 or the system of claim 2, wherein the one or more processors are configured to monitor a response to a diabetes treatment including by

(i) monitoring, and recording a quantitative extent of, a change over time in the shape of the curvilinear probability density function in a direction of being more Gaussian; and
(ii) monitoring, and recording a quantitative extent of, a shift of the curvilinear probability density function reflecting a lowering of a glucose level at which one or more peaks appear in the curvilinear probability density function.

14. The method of claim 1 or the system of claim 2, wherein the one or more processors are configured to output at least one of

(i) a type of diabetes, an existence of hyperglycemia, an existence of hypoglycemia, and a degree of severity of the diabetes;
(ii) a tentative diagnostic suggestion; and

(iii) a tentative treatment suggestion.

15. The method of claim 1 or the system of claim 2, wherein the multiple times of each day in the initial time period includes at least a first time before a meal and a second time after a meal.

16. The method of claim 1 or the system of claim 2, wherein the constructing, using the one or more processors, of the personalized target curvilinear probability density function for the patient is also based on the quantitative assessment.

**Figure 1A**

**Figure 1B**

**Figure 1C**

**Figure 2D**

Figure 2A

Figure 2B

Figure 2C

**Figure 3A**

**Figure 3B**

Figure 4A

Figure 4B

**Figure 5**

**Summary Statistics**

| | |
|---|---|
| Mean | 192.42623 |
| Std Dev | 25.557234 |
| N | 122 |
| Minimum | 153 |
| Maximum | 294 |
| Median | 193 |

**Summary Statistics**

| | |
|---|---|
| Mean | 233.00877 |
| Std Dev | 27.218063 |
| N | 114 |
| Minimum | 157 |
| Maximum | 281 |
| Median | 238 |

**Summary Statistics**

| | |
|---|---|
| Mean | 235.29565 |
| Std Dev | 24.341133 |
| N | 115 |
| Minimum | 150 |
| Maximum | 274 |
| Median | 241 |

**Figure 6A**

COG & Mean(COG) vs. 2 hr increments

Each error bar is constructed using 1 standard deviation from the mean.

**Figure 6B**

**Figure 6C**

**Figure 6D**

METHOD - 100

MEASURING MOMENTARY GLUCOSE LEVELS OF THE PATIENT MULTIPLE TIMES A DAY FOR AT LEAST A WEEK

—110

RECORDING THE MOMENTARY GLUCOSE LEVELS WITH OPTIONAL INFORMATION ASSOCIATED WITH THE RECENT GLUCOSE READINGs

—120

CONSTRUCTING CURRENT CURVILINEAR PROBABILITY DENSITY FUNCTIONS OF OCCURRENCES OF THE MOMENTARY GLUCOSE LEVELS THROUGHOUT THE INITIAL TIME PERIOD AND DISPLAYING THEM

130

DETERMINING A QUANTITATIVE ASSESSMENT OF AT LEAST ONE OF (I) INSULIN RESISTANCE, (II) GLUCOSE VARIABILITY, (III) GLUCOSE SEVERITY INDEX OR (IV) GLUCOSE BURDEN, DURING THE MEASUREMENT PERIOD

140

CONSTRUCTING, USING THE ONE OR MORE PROCESSORS, A PERSONALIZED TARGET CURVILINEAR PROBABILITY DENSITY FUNCTION FOR THE PATIENT AND DISPLAYING IT

150

**FIG. 7A**

METHOD - 100

MEASURING MOMENTARY GLUCOSE LEVELS OF THE PATIENT MULTIPLE TIMES A DAY FOR AT LEAST A WEEK

110

RECORDING THE MOMENTARY GLUCOSE LEVELS WITH OPTIONAL INFORMATION ASSOCIATED WITH THE RECENT GLUCOSE READINGS

120

CONSTRUCTING CURRENT CURVILINEAR PROBABILITY DENSITY FUNCTIONS OF OCCURRENCES OF THE MOMENTARY GLUCOSE LEVELS THROUGHOUT THE INITIAL TIME PERIOD AND DISPLAYING THEM

130

DETERMINING A QUANTITATIVE ASSESSMENT OF INSULIN RESISTANCE DURING THE MEASUREMENT PERIOD

140

DETERMINING A QUANTITATIVE ASSESSMENT OF GLUCOSE VARIABILITY DURING THE MEASUREMENT PERIOD

150

DETERMINING A QUANTITATIVE ASSESSMENT OF GLUCOSE BURDEN DURING THE MEASUREMENT PERIOD

160

DETERMINING A QUANTITATIVE ASSESSMENT OF GLUCOSE SEVERITY INDEX DURING THE MEASUREMENT PERIOD

170

CONSTRUCTING, USING THE ONE OR MORE PROCESSORS, A PERSONALIZED TARGET CURVILINEAR PROBABILITY DENSITY FUNCTION FOR THE PATIENT AND DISPLAYING IT

180

**FIG. 7B**

**Figure 8**

**Figure 9**

**Figure 10**

**Figure 11**

**Figure 12**

**Figure 13**

**FIG. 14**

Figure 15

Figure 16

Occurrence

50

25

10

0

50  80   100..........175....................300........400

Glucose Level

Figure 17

Occurrence

50

25

10

5

50  80   100.................................300.......400

Glucose Level

Figure 18

Europäisches
Patentamt

European
Patent Office

Office européen
des brevets

# EUROPEAN SEARCH REPORT

Application Number

EP 19 18 8731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/128023 A1 (RIBACK JACOB LARS FREDRIK [SE] ET AL) 11 May 2017 (2017-05-11) * paragraphs [0134], [0168], [0207], [0213], [0224]; figures 1,13B * ----- | 1-16 | INV. A61B5/145 |
| A | US 2011/245634 A1 (RAY PINAKI [US] ET AL) 6 October 2011 (2011-10-06) * paragraphs [0075], [0078], [0125] * ----- | 1-16 | |

**TECHNICAL FIELDS SEARCHED (IPC)**

A61B

The present search report has been drawn up for all claims

| Place of search | Date of completion of the search | Examiner |
|---|---|---|
| The Hague | 20 November 2019 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 18 8731

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2019

| Patent document cited in search report | | Publication date | Patent family member(s) | | Publication date |
|---|---|---|---|---|---|
| US 2017128023 | A1 | 11-05-2017 | CA | 2770564 A1 | 17-02-2011 |
| | | | CA | 2770581 A1 | 17-02-2011 |
| | | | CA | 2770591 A1 | 17-02-2011 |
| | | | CN | 102549587 A | 04-07-2012 |
| | | | CN | 102549588 A | 04-07-2012 |
| | | | CN | 102576381 A | 11-07-2012 |
| | | | EP | 2465057 A1 | 20-06-2012 |
| | | | EP | 2465059 A1 | 20-06-2012 |
| | | | EP | 2465060 A1 | 20-06-2012 |
| | | | JP | 5427951 B2 | 26-02-2014 |
| | | | JP | 5658252 B2 | 21-01-2015 |
| | | | JP | 2013501557 A | 17-01-2013 |
| | | | JP | 2013501558 A | 17-01-2013 |
| | | | JP | 2013501989 A | 17-01-2013 |
| | | | RU | 2012105948 A | 20-09-2013 |
| | | | RU | 2012105949 A | 20-09-2013 |
| | | | RU | 2012105952 A | 20-09-2013 |
| | | | US | 2012203166 A1 | 09-08-2012 |
| | | | US | 2012209091 A1 | 16-08-2012 |
| | | | US | 2012209099 A1 | 16-08-2012 |
| | | | US | 2016193411 A1 | 07-07-2016 |
| | | | US | 2017128023 A1 | 11-05-2017 |
| | | | WO | 2011018460 A1 | 17-02-2011 |
| | | | WO | 2011018464 A1 | 17-02-2011 |
| | | | WO | 2011018465 A1 | 17-02-2011 |
| US 2011245634 | A1 | 06-10-2011 | US | 2011245634 A1 | 06-10-2011 |
| | | | WO | 2011123775 A2 | 06-10-2011 |

EPO FORM P0459

For more details about this annex : see Official Journal of the European Patent Office, No. 12/82